# EUROPEAN PATENT SPECIFICATION

(11) **EP 3 669 915 B1**
(45) Date of publication and mention of the grant of the patent: **11.08.2021**
(21) Application number: 19156518.3
(22) Date of filing: 11.02.2019
(51) Int. Cl.: A61M 15/00, G01N 1/22, A61B 5/00, G01N 15/00

(54) **INHALER CONTROL CLIP AND INHALER CONTROL ARRANGEMENT**
INHALATORSTEUERUNGSCLIP UND INHALATORSTEUERUNGSANORDNUNG
CLIP ET AGENCEMENT DE COMMANDE D'INHALATEUR

(30) Priority: 21.12.2018 EP 18215509
(43) Date of publication of application: 24.06.2020
(73) Proprietor: IHP GmbH - Innovations for High Performance Microelectronics / Leibniz-Institut für innovative Mikroelektronik, 15236 Frankfurt (Oder) (DE)
(72) Inventor: Ortmann, Steffen, 15236 Lebus (DE); Zinke, Benny, 15236 Jacobsdorf (DE)
(74) Representative: Eisenführ Speiser

(56) References cited:
- EP-A2- 3 053 620
- WO-A1-2015/109259
- DE-A1-102016 219 759

## Description

The present invention relates to an inhaler control clip and an inhaler control arrangement.

EP3053620 describes an intelligent inhaler holster including a sensor module to sense various environmental parameters.

DE 10 2016 219 759 A1 describes an inhaler with a sensor unit. When release of an inhalant is triggered by the user, the sensor unit in the inhaler detects, among other quantities, at least one quantity of the ambient atmosphere quantity for transmission to a receiver on the side of an attending physician.

It would be desirable to provide an improved inhaler control clip that allows a user of the inhaler making decisions on measures for protection or treatment of his respiratory tract when the user is exposed to potential health risks that are unrecognizable to the user.

According to a first aspect of the invention an inhaler control clip is proposed. The inhaler control clip comprises:
- a casing comprising a receptacle for accommodating an inhaler;
- a sensor unit arranged in the casing and comprising at least one air pollution sensor, which is configured to perform a measurement of at least one quantity indicative of a concentration of at least one gas component or particulate in an ambient atmosphere and to provide at least one sensor output signal indicative thereof;
- a control unit, which is arranged in the casing, connected with the sensor unit and which is configured to trigger the sensor unit to perform the measurement;
- a communication unit which is arranged in the casing and comprises a communication interface to an external configuration device, and which is configured to receive via the communication interface configuration data indicative of one or more sampling points in time to trigger performance of the measurement and of at least one reference value for ambient atmosphere composition; and
- a warning unit arranged in the casing and configured to receive the sensor output signal and the reference value and to compare the sensor output signal with the reference value and to provide a warning signal upon determining that the concentration of the at least one gas component or particulate in the ambient atmosphere exceeds the reference value; wherein
- the control unit receives the configuration data and is configured to trigger performance of the measurement at configurable sampling points in time using the configuration data.

The sensor unit of the inhaler control clip measures at least one quantity indicative of a concentration of at least one gas component or particulate in an ambient atmosphere. The measurement thus provides information about a user's atmospheric environment. This information provides indications for possible health risks for the respiratory tract of the user of the inhaler that may require measures for protection or treatment.

To allow the user of an inhaler making decisions on such measures while being exposed to that ambient atmosphere, the inhaler control clip has a communication unit comprising a communication interface to an external configuration device. The communication unit is configured to receive via the communication interface configuration data indicative of one or more sampling points in time for triggering performance of the measurement and of at least one reference value for ambient atmosphere composition.

The warning unit of the inhaler control clip provides immediate information upon determining that the concentration of the at least one gas component or particulate in the ambient atmosphere exceeds the reference value, and thus warns the user in case a current quality of the ambient atmosphere forms a health risk.

The capability of the control unit to rigger performance of the measurement at configurable sampling points in time using the configuration data allows dynamically adjusting a frequency of the measurement of the at least one quantity indicative of the concentration of the at least one gas component or particulate in the ambient atmosphere. Based on such an adjusted frequency of measurement, the user receives reliable information for judging the current health risk and for making decisions on prevention or treatment measures, or for instance on moving to another, less polluted area.

The configuration data is provided from an external source. The inhaler control clip of the present invention advantageously provides flexibility in the implementation of such an external source of configuration data, be it in the form of an algorithm running on an external configuration device worn by the user, or from a more remote source, such as an attending physician.

In the following, embodiments of the inhaler control clip will be described.

Exposure to certain pollutants may be bearable for certain patients for a certain amount of time without incurring substantial health risks. In order to allow the user a judgement of a current exposure of this kind, a preferred embodiment of the inhaler control clip has the control unit configured to trigger the sensor unit to perform the measurement at a plurality of sampling points in time within a configurable threshold time span. The warning unit is configured to provide the warning signal, which determines that the concentration of the at least one gas component or particulate in the ambient atmosphere exceeds the reference value at all the sampling points in time within the threshold time span. By making use of configurable threshold times spans, the user obtains increased flexibility for reacting to current stress caused by the ambient atmosphere.

To further increase flexibility for the user in making judgements on health risks involved in a current exposure to an ambient atmosphere, the communication unit of another embodiment of the inhaler control clip is configured to receive via the communication interface a plurality of reference values for the ambient atmosphere composition. The reference values are associated with different gas components or particulates. The warning unit is configured to provide the warning signal upon determining that a combination of at least two of the reference values is exceeded by the concentration of the at least one gas component or particulates in the ambient atmosphere. This allows reacting to more complex pollution scenarios.

Another aspect of increasing flexibility for the user in making judgements on health risks involved in a current exposure to an ambient atmosphere is covered by an embodiment, in which the configuration data additionally provides different combinations of the reference value and the threshold time span for a given gas component or particulate. For instance, a single measurement exceeding a high first reference value of a certain gas component or particulate may trigger a warning signal, just like a series of measurements exceeding a comparatively lower second reference value extending over a longer threshold time span for the same gas component or particulate will trigger the warning signal. The warning unit of this embodiment is configured to determine whether the concentration of the at least one gas component or particulate in the ambient atmosphere exceeds each of the reference values for each of the associated threshold time spans, and to provide the warning signal upon determining that one of the criteria defined by the respective pairs of reference value and assigned threshold time span is fulfilled.

In another embodiment, the configuration data additionally assigns a weight factor to each of the plurality of reference values and associated threshold time spans, preferably based on a configurable setting of user-individual sensitivities to certain gas components or particulates in the ambient atmosphere. This allows the individual user making an appropriate treatment decision in view of his personal sensitivity settings, selectively prioritizing certain pollution scenarios over others.

Regarding the gas components or particulates to be monitored by the sensor unit, the inhaler control clip can be tailored to different application cases. In some embodiments of the inhaler, the air pollution sensor is configured to perform a measurement of a quantity indicative of a concentration of nitrogen dioxide. In other embodiments, it is additional or alternatively configured to perform a measurement of a quantity indicative of a concentration of sulphur dioxide. Additionally or alternatively, the air pollution sensor is configured in another embodiment to perform a measurement of a quantity indicative of a concentration of repairable particulate matter, in particular small particulates such as fine dust. The mentioned quantities are particularly suitable in many cases to determine conditions of an ambient atmosphere exposure, which bear a high risk of impairing the health of the user. Other gas components or particulates can be covered by providing suitable sensors in the sensor unit.

In further embodiments the inhaler control clip additionally comprises at least one ambient sensor, which is configured to perform a measurement of at least one quantity indicative of a temperature of the ambient atmosphere, a relative humidity of the ambient atmosphere or a pressure of the ambient atmosphere. The additional detection of temperature, humidity or pressure of the ambient atmosphere provides additional information regarding risks of an impairment of the health status.

Recording a plurality of the different quantities mentioned herein in the ambient atmosphere of the inhaler control clip is used in some embodiments described further below to determine a multi-dimensional correlation of the different measured quantities, or to determine and provide a site-specific history of complex pollution scenarios.

The warning signal can be provided in many different ways. In some embodiments, the warning signal is an output signal provided for sensual perception by the user. Different examples of this group of embodiments have a warning unit which is configured to provide an optical or an acoustical warning signal, or a combination of an optical and an acoustical warning signal. In some embodiments employing an optical warning signal, the warning unit comprises a light emitting unit configured to emit the warning signal as a light signal in a respective one of a plurality of distinguishable light colors, such as green and red, and to determine the light color to be emitted using an amount of the measured concentration of the at least one gas component or particulate in the ambient atmosphere.

To improve the user experience, preferred embodiments the inhaler control clip comprise a display, which receives the warning signal. The display is configured to provide a graphical representation indicating that the concentration of the at least one gas component or particulate in the ambient atmosphere exceeds the reference value. This way, the presentation of information to the user is particularly flexible and can be enriched by additional information. In one such embodiment, the sensor unit is configured to the display, and the display receives and additionally or alternatively provides a graphical display of a current value of the sensor output signal at a given current sampling point in time. This can be also used as a warning signal, for instance by providing such display of the current value of the sensor output signal only in case the reference value is currently exceeded. In another embodiment of the inhaler control clip, the communication unit is connected with the display and configured to additionally receive via the communication interface payload data that is different from the configuration data, and to forward the payload data to the display. This embodiment allows displaying additional information to the user that has been obtained by sources external to the inhaler control clip, for instance by a configuration device. In one exemplary embodiment of this kind, the communication unit receives and forwards to the display a maximum value of the sensor output signal as determined during a predetermined time span, such as the last hour. In another embodiment of this kind, the payload data received by the communication unit and subsequently displayed on the display is indicative of a recommendation to leave the current ambient atmosphere.

In an embodiment of the inhaler control clip the control unit is configured to trigger the communication unit to perform a configuration exchange sequence with the external configuration device. The configuration exchange sequence comprises forwarding the sensor output signal to the configuration device and receiving in response updated configuration data from the external configuration device. In this case, the communication unit is configured to perform the configuration exchange sequence and forward the received updated configuration data to the control unit. The configuration exchange sequence allows a dynamic and automatic adaptation of the sampling points in time to be used by the inhaler control clip.

In one embodiment, the inhaler control clip further comprises:
- a scanning unit configured to scan a surface section of the container to detect a graphical code identifying a type of medication in the storage container and provide a scanning unit output signal indicative thereof.

In this embodiment, the configuration exchange sequence further comprises providing the scanning unit output signal to the external configuration device.

The graphical code is, for instance, a bar code or a QR code applied on an outer surface of the storage container. This is useful in particular where the inhaler control clip allows a user to insert different storage containers comprising different types of medication. Based on information regarding the currently applied medication, an adaptation of the configuration data can be made by the external configuration device, for example regarding the sampling points in time for triggering performance of measurements. For example, a medical inhalant for long-term medication is detected, which results in scheduling measurements with regular sampling periods having a comparatively large time span between individual sampling points, e.g., having one sampling point in time per hour. In another example, a medical inhalant for rescue medication (reliever) is detected. In this case, preferably after release of the inhalant it is suitable to increase a number of the sampling points in time for documenting the effects of the medication in view of the composition of the ambient atmosphere, e.g., once every 5 minutes.

To further improve the inhaler control clip, the communication unit of one embodiment is configured to receive via the communication interface medication recommendation data indicative of a recommended type of medication or medication dosage or a combination of these types of data, and to provide the medication recommendation data to the display for output to a user. This allows a user-individual medication.

In a non-limiting exemplary embodiment, an algorithm running on an external configuration device receives the sensor output signal and the scanning unit output signal and determines the medication recommendation data. In another exemplary embodiment, an attending physician provides the medication recommendation data to the communication unit of the inhaler, based on received scanning unit output signals and sensor unit output signals.

In an embodiment the inhaler control clip comprises an actuation sensor, which is configured to detect actuation of an inhalation actuating element of an external inhaler that has been inserted into the inhaler control clip, i.e., is in an inserted state, and to provide an actuation signal indicative thereof. The control unit of this embodiment is preferably configured to trigger the sensor unit to perform the measurement upon receiving the actuation signal. The actuation signal provided by the actuation sensor is in some embodiments a signal pertaining to a measurement of a physical quantity, and is for example, provided by a pressure sensor and thus indicative of a pressure exerted by a user of the inhaler, which reaches a known calibrated value range for actuation. In another example, the actuation signal is derived from an acoustical measurement, for instance obtained by a microphone.

In other embodiments, the inhaler control clip comprises a measurement actuation element, actuation of which enables a manual triggering of the sensor unit by the user of the inhaler control clip, e.g. ,when the user feels uncomfortable or sick. Thus, the inhaler control clip allows triggering additional measurements on demand to get information of the current atmospheric characteristics on-site.

According to a second aspect of the invention inhaler control arrangement is proposed. The inhaler control arrangement comprises:
- at least one inhaler according to an embodiment of the first aspect of the invention, wherein
   - the control unit is configured to trigger the communication unit to perform a configuration exchange sequence with the external configuration device, the configuration exchange sequence comprising forwarding the sensor output signal to the configuration device and receiving in response updated configuration data from the external configuration device; and wherein
   - the communication unit is configured to perform the configuration exchange sequence and forward the received updated configuration data to the control unit.

The inhaler control arrangement further comprises a configuration device communicatively connectable to the communication unit of the inhaler and comprising
- a configuration communication interface, which is configured to receive one or more of the sensor output signals from the communication unit of the inhaler and to provide updated configuration data to the communication unit of the inhaler;
- an evaluation unit configured to determine, using the received one more sensor signals, updated configuration data to be used by the inhaler and to provide the updated configuration data to the configuration communication interface.

The configuration device provides and maintains configuration data, in particular reference values and sampling points in time, preferably also threshold time spans and medication doses, depending on the respective embodiment of the inhaler used.

In preferred embodiments, the configuration device is a portable computer to be worn by the user of the inhaler, such as a smart phone, tablet computer, or notebook computer. In these embodiments, the configuration exchange sequence is preferably transmitted between the configuration device and the inhaler via a radio communication suitable for local communication, for example via a ZigBee, WiFi (WLAN) or Bluetooth connection. In other embodiments of the inhaler control arrangement the external configuration device is a standard desktop computer or a computer server to be arranged remotely from the user. In these embodiments, the configuration exchange sequence is preferably transmitted between the configuration device and the inhaler via radio communication suitable for long-distance communication, for example via a cellular radio communication.

In an embodiment of the inhaler control arrangement the evaluation unit of the configuration device is configured to assign time stamps to incoming sensor output signals and to determine and store a pollution profile indicative of the concentration of the at least one gas component or particulate in the ambient atmosphere as a function of time during a predetermined monitoring time span. The pollution profile can be used for analysis of the pollution to which the user has been exposed, and, based on this analysis, for determining additional treatment or future prevention measures. In another embodiment of the inhaler control arrangement, the evaluation unit of the configuration device is configured to determine the updated configuration data in dependence on the pollution profile.

Recording a plurality of the different quantities as a function of time is suitable for determining a multi-dimensional correlation of the different measured quantities. In some embodiments of the mobile configuration device, a location sensor such as a GPS receiver unit is additionally provided, preferably in the configuration device, for determining a position of the user. The evaluation unit is further configured to assign geographic location data to a received sensor output signal. Suitably, the evaluation unit is further configured to determine location-specific pollution history data from the sensor output signals associated with a given location, as defined by its geographic location data. This is used in some embodiments to determine locations the user should avoid, for instance when using the configuration device for determining outdoor routes for the user.

In the following, further embodiments will be described with reference to the enclosed drawings. In the drawings:
Fig. 1 shows a schematic block diagram of an embodiment of an inhaler control clip;
Fig. 2a shows a perspective view of another embodiment of an inhaler control clip as seen from one side;
Fig. 2b shows a perspective view of the inhaler control clip of Fig. 2a from another side;
Fig. 2c shows a perspective view of the inhaler control clip of Figs. 2a and 2b with an inserted inhaler.
Fig. 3 shows a schematic block diagram of an embodiment of an inhaler control clip control arrangement; and
Fig. 4 shows a pollution profile determined using an inhaler control arrangement of Fig. 3.

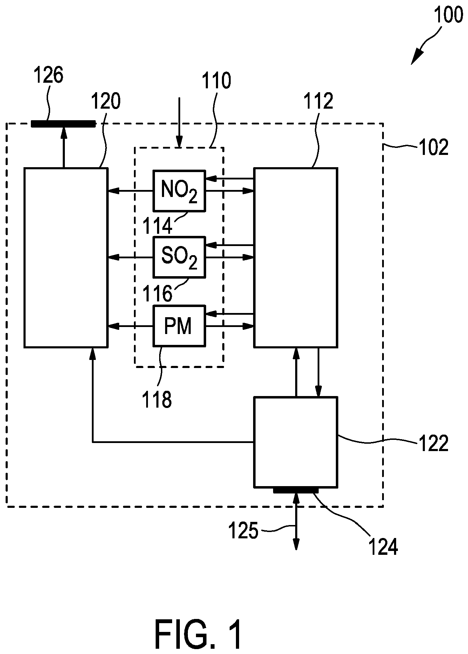

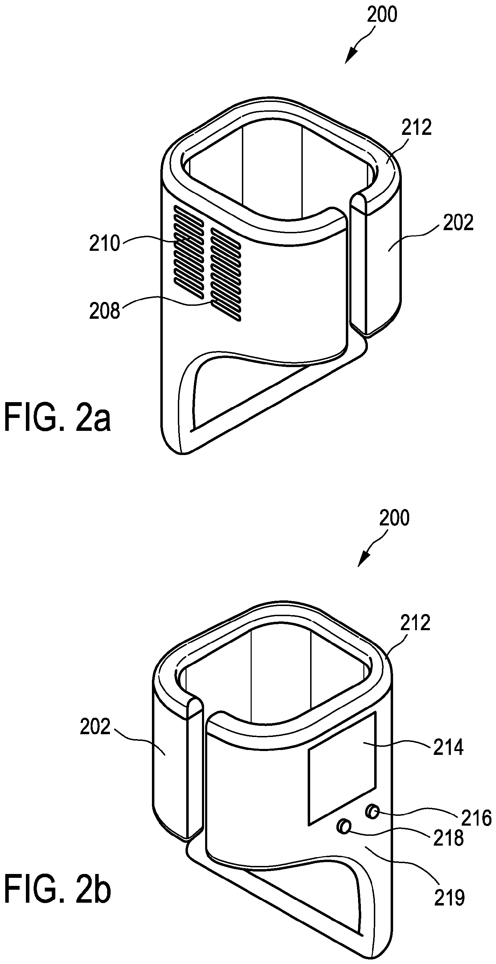

Fig. 1 shows a schematic block diagram of an embodiment of an inhaler control clip 100. The inhaler control clip 100 comprises a casing 102 with a receptacle for accommodating an inhaler that is an external device with respect to the inhaler control clip 100. The receptacle is not shown in Fig. 1, but will be shown in further examples in Fig. 2a and 2b.

An inhalant in the inhaler that can be inserted into the inhaler control clip 100 is for example a medical inhalant for long-term medication of a user, e.g., for use as an asthma spray containing corticosteroids. The inhalant is in other examples a medical inhalant for rescue medication, containing, e.g., beta-sympathomimetics.

A sensor unit 110 in the inhaler control clip 100 is arranged in the casing 102 and in the present embodiment has three air pollution sensors 114,116 and 118. The number of air pollution sensors can be higher or lower, and is to be selected according to the requirements of a given application case. In the present example, the three air pollution sensors 114,116 and 118 are configured to perform a measurement of a concentration of nitrogen dioxide NO₂, sulphur dioxide SO₂ and fine dust PM, respectively, in an ambient atmosphere. Each of the air pollution sensors 114, 116, 118 provides a respective sensor output signal.

The sensor unit 110 is connected to a control unit 112, which is configured to trigger the sensor unit to perform the measurement at a plurality of sampling points in time within a configurable threshold time span. The control unit 112 also receives the sensor output signals from the sensor unit 110.

A communication unit 122 has a communication interface 124 is included in the casing 102 of the inhaler control clip 100. The communication unit 122 receives, via the communication interface 124, configuration data from an external configuration device (not shown in Fig. 1), as indicated by an incoming direction of double arrow 125. The configuration data are indicative of sampling points in time to trigger performance of the measurement. The configuration data can provide the sampling points in time, hereinafter also called sampling times, in different ways. In one example, the configuration data is indicative of a time span between sampling times, or of a sampling frequency. Furthermore, the configuration data comprises reference values for the gas components nitrogen dioxide NO₂, sulphur dioxide SO₂ and for fine dust PM concentration. An exemplary reference value for NO₂ is 40 ppb and an exemplary reference value for SO₂ is 20 ppb. An exemplary reference value for fine dust of a size falling under the PM₁₀ classification is 20 µg/m³.

The communication unit 122 forwards the received configuration data to the control unit 112.

The warning unit 120 receives the reference values and the sensor output signals. The warning unit compares the sensor unit output signals to the respective reference values. If the respective sensor unit output signal exceeds an associated respective reference value, a warning signal is generated and provided by the warning unit 120.

In the present embodiment, the warning unit 120 transmits the warning signal to a light emitting unit 126. Light emitted by the light emitting unit 126 indicates an ambient atmosphere having air pollutant concentrations falling below the reference values with a green light and an ambient atmosphere having air pollutant concentrations higher than the reference values with a red light.

In the following, optional additional functionalities of the inhaler control clip 100 will be described, still with reference to Fig. 1.

In one variant, the control unit 112 triggers the communication unit 122 to perform a configuration exchange sequence with an external configuration device (not shown in Fig. 1), as indicated by the double arrow 125 in Fig. 1. The configuration exchange sequence comprises forwarding the sensor output signals of the individual air pollution sensors to the external configuration device, and receiving in response updated configuration data from the external configuration device. The updated configuration data for instance comprises an updated frequency of sampling points in time, determined by the external configuration device using the sensor output signals of the respective air pollution sensors received from the inhaler control clip 100.

In another variant, the warning unit 120 additionally provides a warning signal, if the sensor unit output signal exceeds the reference value at all the sampling points in time within a threshold time span. For example, the warning signal is provided when the sensor unit output signal of the NO₂ sensor indicates concentrations having exceeded a reference value of 10 ppb for a minimum of 60 minutes, which forms the threshold time span. In another example the warning signal is provided, when the sensor unit output signal of the NO₂ sensor indicates that the concentration has exceeded 20 ppb for a minimum of 15 minutes and the sensor unit output signal of the SO₂ sensor indicates that the concentration has exceeded 10 ppb for the same reference time span of 15 minutes.

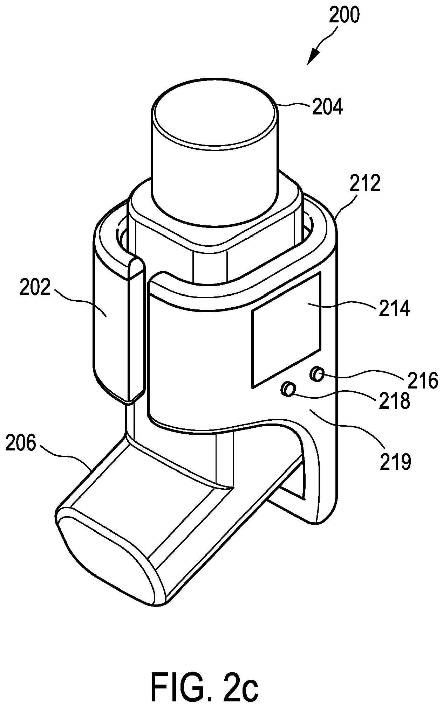

Fig. 2a and b show two perspective views of another embodiment of an inhaler control clip 200 from two sides. Fig. 2c shows a perspective view of the inhaler control clip of Figs. 2a and 2b with an inserted inhaler 204 having a mouthpiece 206. The following description will refer to all three figures in parallel. The inhaler control clip 200 has a casing 202 for accommodating an inhaler. The casing 202 of the inhaler control clip 200 has a clamp shape and is made of a material with suitable elastic properties to resiliently clamp an inhaler 204 inside the casing 202. For instance, certain plastics are suitable materials. Of course, other materials like metals and other geometries can be used for the casing. Fixation of the inhaler 204 inside the inhaler control clip 200 may also be achieved by providing a form-fit that removably locks the inhaler 204 inside the inhaler control clip in an inserted state.

In this exemplary embodiment, the inhaler control clip 200 has two air pollution sensors, not shown here, inside the casing 202. The air pollution sensors measure different gas components in ambient atmosphere, for example NO₂ and SO₂. Openings 208 and 210 in the casing 202 formed by a plurality of slits are arranged allow access of the ambient atmosphere to the air pollution sensors. Furthermore, the inhaler control clip 200 comprises, inside the casing 202 and therefore not visible in Figs. 2a and 2b, a sensor unit, a control unit and a communication unit, whose functionality corresponds to the one of the exemplary inhaler control clip 100, inside the casing 202. A warning unit comprising a LED 212 is arranged on the casing 202. The LED has the same functionality as the light emitting unit of the exemplary inhaler control clip 100.

As can be seen in Fig. 2b a display 214 is arranged on one side surface 219 of the inhaler control clip 200. The display 214, additionally to the LED 212, displays measurement information generated by the air pollution sensors. For instance, the display is configured to provide a graphical representation indicating that the sensor unit output signal of nitrogen dioxide NO₂, sulphur dioxide SO₂ or fine dust PM is exceeding the respective reference value. Such display can also provide information which of the individual reference values is exceeded.

Furthermore, two control buttons 216 and 218 are arranged for actuation by the user of the inhaler control clip 200. Actuation of the control button 216 triggers the sensor unit to perform the measurement. Actuation of the control button 218 changes between display view modes, for example between a warning display shown current warning signals, and sensor signal display of either NO₂ or SO₂ or battery status.

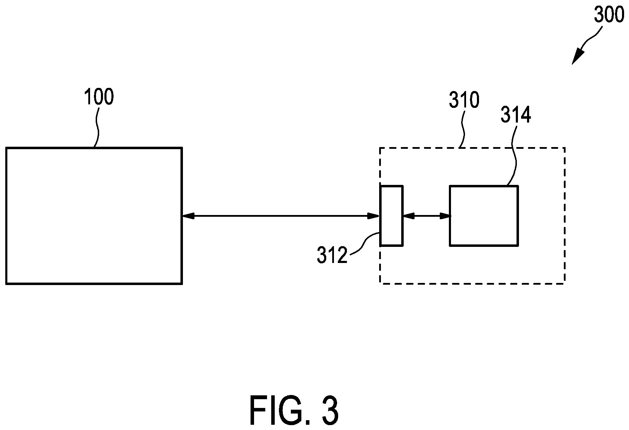

Fig. 3 shows a schematic block diagram of an embodiment of an inhaler control arrangement 300. The inhaler control arrangement 300 is used to support the monitoring of potential health risks, for example of an asthma patient.

The inhaler control arrangement 300 comprises the inhaler control clip 100 of Fig. 1 and a configuration device 310. Thus, for a description of the inhaler control clip 100, reference is made to the description of Fig. 1 hereinabove. The following description focusses on the configuration device 310.

The configuration device 310 comprises a configuration communication interface 312 and an evaluation unit 314. The configuration device 310 in for example implemented as a software application that is executable by a portable computer such as smart phone. The software application (app) is designed to allow the smart phone receiving and evaluating the sensor output signals, i.e., implement the communication interface 312 and the evaluation 314. Based on the evaluation performed by the evaluation unit 314, configuration data to be used by the inhaler control clip is updated. The configuration device 310 is connected via its configuration communication interface 312 to the communication unit 122 of the inhaler control clip 100. The updated configuration data is provided via the configuration communication interface to the communication unit 122.

Additionally, the evaluation unit 314 assigns the incoming sensor output signals with time stamps and determines and stores a pollution profile indicative of the concentration of the measured gas components or particulates, for example nitrogen dioxide NO₂ and sulphur dioxide SO₂. The updated configuration data can be determining in dependence on the pollution profile.

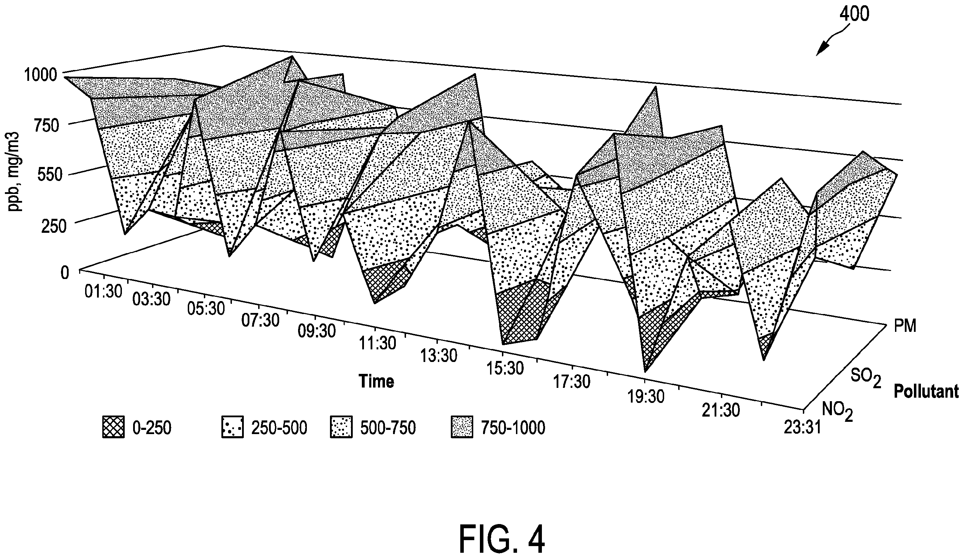

Fig. 4 shows an exemplarily pollution profile 400 determined by the evaluation unit 314. In the pollution profile the determined concentration values of nitrogen dioxide NO₂, sulphur dioxide SO₂ and fine dust PM are shown as a function of time. The pollution profile 400 is plotted in a three dimensional coordinate system. The x-axis indicates the time over a time span of 24 hours. The y-axis indicates the concentration of the individual pollutants in units of ppb for NO₂ and SO₂ and in units of mg/m³ for PM. The concentration values of the individual pollutants, here NO₂, SO₂ and PM, are plotted for the respective sampling times at different z-axis positions. In this exemplary embodiment, one sampling point is plotted per hour. The pollution profile uses different colors or other graphical distinctions for classifying the concentration values into different concentration intervals, based on predetermined reference values.

The pollution profile 400 is used for complex analysis of the sensor output signals. For example, structures of specific parts in the pollution profile can be extracted and used as a search template for similar structures. These structures in the pollution profile can be, for instance, correlated with a potential health risk for the user of the inhaler control clip. Thus, the complex analysis of the pollution profile can be used for treatment or future prevention measures.

In summary, an inhaler control clip comprises a sensor unit with an air pollution sensor, which measures at least one quantity indicative of a concentration of a gas component or particulate in an ambient atmosphere. A control unit triggers the sensor unit to perform the measurement. A communication unit has a communication interface to an external configuration device, and receives configuration data indicative sampling points in time to trigger performance of the measurement, and of at least one reference value for ambient atmosphere composition. The control unit receives the configuration data and is configured to trigger performance of the measurement at configurable sampling points in time using the configuration data. A warning unit receives the sensor output signal and the reference value and compares the sensor output signal with the reference value, and provides a warning signal upon determining that the concentration of the gas component or particulate exceeds the reference value.

## Claims

1. An inhaler control clip (100, 200) comprising
- a casing (102) comprising a receptacle for accommodating an inhaler;
- a sensor unit (110) arranged in the casing and comprising at least one air pollution sensor (114, 116, 118), which is configured to perform a measurement of at least one quantity indicative of a concentration of at least one gas component or particulate in an ambient atmosphere and to provide at least one sensor output signal indicative thereof;
- a control unit (112), which is arranged in the casing (102), connected with the sensor unit (110) and which is configured to trigger the sensor unit (110) to perform the measurement;
- a communication unit (122) which is arranged in the casing and comprises a communication interface (124) to an external configuration device, and which is configured to receive via the communication interface configuration data indicative of one or more sampling points in time to trigger performance of the measurement and of at least one reference value for ambient atmosphere composition; and
- a warning unit (120) arranged in the casing (102) and configured to receive the sensor output signal and the reference value and to compare the sensor output signal with the reference value and to provide a warning signal upon determining that the concentration of the at least one gas component or particulate in the ambient atmosphere exceeds the reference value; wherein
- the control unit (112) receives the configuration data and is configured to trigger performance of the measurement at configurable sampling points in time using the configuration data.

2. An inhaler control clip (100, 200) according to claim 1, wherein
- the control unit (112) is configured to trigger the sensor unit (110) to perform the measurement at a plurality of sampling points in time within a configurable threshold time span; and wherein
- the warning unit (120) is configured to provide the warning signal upon determining that the concentration of the at least one gas component or particulate in the ambient atmosphere exceeds the reference value at all the sampling points in time within the threshold time span.

3. An inhaler control clip (100, 200) according to claim 1 or 2,
- the communication unit (122) is configured to receive via the communication interface (124) a plurality of reference values for the ambient atmosphere composition that are associated with different gas components or particulates;
- the warning unit (120) is configured to provide the warning signal upon determining that a combination of at least two of the reference values is exceeded by the concentration of the at least one gas component or particulates in the ambient atmosphere.

4. An inhaler control clip (100, 200) according one of the preceding claims, wherein the air pollution sensor (114, 116, 118) is configured to perform a measurement of a quantity indicative of a concentration of nitrogen dioxide or sulphur dioxide.

5. An inhaler control clip (100, 200) according to one of the preceding claims, further comprising at least one ambient sensor which is configured to perform a measurement of at least one quantity indicative of a temperature of the ambient atmosphere, a relative humidity of the ambient atmosphere or a pressure of the ambient atmosphere.

6. An inhaler control clip (100, 200) according to one of the preceding claims, further comprising a display (214), which receives the warning signal and is configured to provide, upon receiving the warning signal, a graphical representation indicating that the concentration of the at least one gas component or particulate in the ambient atmosphere exceeds the reference value.

7. An inhaler control clip (100, 200) according to one of the preceding claims, wherein the warning unit comprises a light emitting unit (126, 212) configured to emit the warning signal as a light signal in a respective one of a plurality of distinguishable light colors, and to determine the light color to be emitted using an amount of the measured concentration of the at least one gas component or particulate in the ambient atmosphere.

8. An inhaler control clip (100, 200) according to one of the preceding claims, wherein
- the control unit (112) is configured to trigger the communication unit (122) to perform a configuration exchange sequence (125) with the external configuration device, the configuration exchange sequence (125) comprising forwarding the sensor output signal to the external configuration device and receiving in response updated configuration data from the external configuration device; and wherein
- the communication unit (122) is configured to perform the configuration exchange sequence and forward the received updated configuration data to the control unit (112).

9. An inhaler control clip (100, 200) according to claim 8, further comprising
- a scanning unit configured to scan a surface section of the container to detect a graphical code identifying a type of medication in the storage container and provide a scanning unit output signal indicative thereof;
wherein the configuration exchange sequence further comprises providing the scanning unit output signal to the external configuration device.

10. An inhaler control clip (100, 200) according to claim 6, wherein the communication unit (122) is further configured to receive via the communication interface medication recommendation data indicative of a recommended medication dosage and to provide the medication recommendation data to the display for output to a user.

11. An inhaler control clip (100, 200) according to one of the preceding claims, further comprising an actuation sensor, which is configured to detect actuation of an inhalating actuating element on an external inhaler (204) in an inserted state and to provide an actuation signal indicative thereof, wherein the control unit (112), upon receiving the actuation signal, is configured to trigger the sensor unit (110) to perform the measurement.

12. An inhaler control arrangement (300) comprising
- at least one inhaler control clip (100) according to claim 1;
- a configuration device (310) communicatively connectable to the communication unit (122) of the inhaler control clip (100) and comprising
- a configuration communication interface (312), which is configured to receive one or more of the sensor output signals from the communication unit (122) of the inhaler control clip (100) and to provide updated configuration data to the communication unit (122) of the inhaler control clip;
- an evaluation unit (314) configured to determine, using the received one or more sensor signals, updated configuration data to be used by the inhaler control clip (100) and to provide the updated configuration data to the configuration communication interface (312).

13. An inhaler control arrangement (300) according to claim 12, wherein the evaluation unit (314) of the configuration device (310) is configured
- to assign time stamps to incoming sensor output signals and to determine and store a pollution profile (400) indicative of the concentration of the at least one gas component or particulate in the ambient atmosphere as a function of time during a predetermined monitoring time span.

14. An inhaler control arrangement (300) according to claim 13, wherein the evaluation unit (314) of the configuration device (310) is configured to determine the updated configuration data in dependence on the pollution profile (400).

## Patentansprüche

1. Inhalatorsteuerclip (100, 200) aufweisend
- ein Gehäuse (102) aufweisend eine Aufnahme zum Aufnehmen eines Inhalators;
- eine Sensoreinheit (110), die in dem Gehäuse angeordnet ist und wenigstens einen Luftverschmutzungssensor (114, 116, 118) aufweist, der ausgebildet ist, eine Messung von wenigstens einer Größe durchzuführen, die indikativ für eine Konzentration wenigstens einer Gaskomponente oder Partikel in einer Umgebungsatmosphäre ist, und wenigstens ein diese anzeigendes Sensorausgangssignal bereitzustellen;
- eine Steuereinheit (112), die in dem Gehäuse (102) angeordnet, mit der Sensoreinheit verbunden und ausgebildet ist, die Sensoreinheit (110) dazu zu veranlassen, die Messung durchzuführen;
- eine Kommunikationseinheit (112), die in dem Gehäuse angeordnet ist und eine Kommunikationsschnittstelle (124) zu einem externen Konfigurationsgerät aufweist, und die ausgebildet ist, über die Kommunikationsschnittstelle Konfigurationsdaten zu empfangen, die indikativ sind für einen oder mehrere Abtastzeitpunkte, um ein Durchführen der Messung und zu veranlassen, und für wenigstens einen Referenzwert für eine Umgebungsatmosphärenzusammensetzung; und
- eine Warneinheit (120), die in dem Gehäuse (102) angeordnet und ausgebildet ist, das Sensorausgangssignal und den Referenzwert zu empfangen und das Sensorausgangssignal mit dem Referenzwert zu vergleichen und ein Warnsignal bereitzustellen, falls ermittelt wird, dass die Konzentration der wenigstens einen Gaskomponente oder Partikel in der Umgebungsatmosphäre den Referenzwert übersteigt; wobei
- die Steuereinheit (112) die Konfigurationsdaten empfängt und ausgebildet ist, die Konfigurationsdaten zu verwenden, um ein Durchführen der Messung zu konfigurierbaren Abtastzeitpunkten zu veranlassen.

2. Inhalatorsteuerclip (100, 200) gemäß Anspruch 1, wobei
- die Steuereinheit (112) ausgebildet ist, die Sensoreinheit dazu zu veranlassen, die Messung zu einer Vielzahl von Abtastzeitpunkten innerhalb einer konfigurierbaren Schwellenwertzeitspanne durchzuführen; und wobei
- die Warneinheit (120) ausgebildet ist, das Warnsignal bereitzustellen, falls ermittelt wird, dass die Konzentration der wenigstens einen Gaskomponente oder Partikel in der Umgebungsatmosphäre den Referenzwert an allen der Abstastzeitpunkte innerhalb der Schwellenwertzeitspanne übersteigt.

3. Inhalatorsteuerclip (100, 200) gemäß Anspruch 1 oder 2, wobei
- die Kommunikationseinheit (122) ausgebildet ist, über die Kommunikationsschnittstelle (124) eine Vielzahl von Referenzwerten für die Umgebungsatmosphärenzusammensetzung zu empfangen, die unterschiedlichen Gaskomponenten oder Partikeln in der Umgebungsatmosphäre zugeordnet sind;
- die Warneinheit (120) ausgebildet ist, das Warnsignal bereitzustellen, falls ermittelt wird, dass eine Kombination aus wenigstens zwei Referenzwerten durch die Konzentration der wenigstens einen Gaskomponente oder Partikel in der Umgebungsatmosphäre überstiegen wird.

4. Inhalatorsteuerclip (100, 200) gemäß einem der vorstehenden Ansprüche, wobei der Luftverschmutzungssensor (114, 116, 118) ausgebildet ist, eine Messung von einer Größe durchzuführen, die indikativ für eine Konzentration von Stickstoffdioxid oder Schwefeldioxid ist.

5. Inhalatorsteuerclip (100, 200) gemäß einem der vorstehenden Ansprüche, weiterhin aufweisend wenigstens einen Umgebungssensor, der ausgebildet ist, eine Messung von wenigstens einer Größe durchzuführen, die indikativ für eine Temperatur der Umgebungsatmosphäre, eine relative Feuchtigkeit der Umgebungsatmosphäre oder eines Druckes der Umgebungsatmosphäre ist.

6. Inhalatorsteuerclip (100, 200) gemäß einem der vorstehenden Ansprüche, weiterhin aufweisend eine Anzeige (214), die das Warnsignal empfängt und ausgebildet ist, nach Empfang des Warnsignals, eine graphische Darstellung bereitzustellen, die angibt, dass die Konzentration der wenigstens einen Gaskomponente oder Partikel in der Umgebungsatmosphäre den Referenzwert übersteigt.

7. Inhalatorsteuerclip (100, 200) gemäß einem dervorstehenden Ansprüche, wobei die Warneinheit eine Lichtemissionseinheit (126, 212) aufweist, die ausgebildet ist, das Warnsignal als ein Lichtsignal in einer jeweiligen von einer Vielzahl unterschiedlicher Lichtfarben zu emittieren, und eine Menge der gemessenen Konzentration der wenigstens einer Gaskomponente oder Partikel in der Umgebungsatmosphäre zu verwenden, um die zu emittierenden Lichtfarbe zu bestimmen.

8. Inhalatorsteuerclip (100, 200) gemäß einem der vorstehenden Ansprüche, wobei
- die Steuereinheit (112) ausgebildet ist, die Kommunikationseinheit (122) dazu zu veranlassen, eine Konfigurationsaustauschabfolge (125) mit dem externen Konfigurationsgerät durchzuführen, wobei die Konfigurationsaustauschabfolge (125) ein Weiterleiten des Sensorausgangssignals an das externe Konfigurationsgerät und, in Antwort darauf, ein Empfangen aktualisierter Konfigurationsdaten von dem externen Konfigurationsgerät umfasst, und wobei
- die Kommunikationseinheit (122) ausgebildet ist, die Konfigurationsaustauschabfolge durchzuführen und die aktualisierten Konfigurationsdaten an die Steuereinheit (112) weiterzuleiten.

9. Inhalatorsteuerclip (100, 200) gemäß Anspruch 8, weiterhin aufweisend
- eine Scaneinheit, die ausgebildet ist, einen Oberflächenabschnitt eines Behälters zu scannen, um einen graphischen Code zu erfassen, der eine Art von Arzneimittel in dem Aufbewahrungsbehälter kennzeichnet und ein dieses anzeigendes Scaneinheitausgangssignal bereitzustellen;
wobei die Konfigurationsaustauschabfolge weiterhin ein Bereitstellen des Scaneinheitausgangssignals an das externe Konfigurationsgerät umfasst.

10. Inhalatorsteuerclip (100, 200) gemäß Anspruch 6, wobei die Kommunikationseinheit (122) weiterhin ausgebildet ist, über die Kommunikationsschnittstelle Arzneimittelempfehlungsdaten zu empfangen, die für eine empfohlene Arzneimitteldosis indikativ sind, und die Arzneimittelempfehlungsdaten an die Anzeige zur Ausgabe an einen Nutzer bereitzustellen.

11. Inhalatorsteuerclip (100, 200) gemäß einem der vorstehenden Ansprüche, weiterhin aufweisend einen Betätigungssensor, der ausgebildet ist, ein Betätigen eines Inhalationsbetätigungselements an einem externen Inhalator (204) in einem eingesetzten Zustand zu erfassen und ein diese anzeigendes Betätigungssignal bereitzustellen, wobei die Steuereinheit (112) ausgebildet ist, nach Empfang des Betätigungssignals die Sensoreinheit (110) dazu zu veranlassen die Messung durchzuführen.

12. Inhalatorsteueranordnung (300) aufweisend
- wenigstes einen Inhalatorsteuerclip (100) gemäß Anspruch 1;
- ein Konfigurationsgerät (310), das mit der Kommunikationseinheit (122) des Inhalatorsteuerclips (100) kommunizierend verbindbar ist und aufweist
- eine Konfigurationskommunikationsschnittstelle (312), die ausgebildet ist, eines oder mehrere Sensorausgangssignale von der Kommunikationseinheit (122) des Inhalatorsteuerclips (100) zu empfangen und aktualisierte Konfigurationsdaten an die Kommunikationseinheit (122) des Inhalatorsteuerclips bereitzustellen;
- eine Auswerteeinheit (314), die ausgebildet ist, das eine oder die mehreren empfangenen Sensorsignale dazu zu verwenden, von dem Inhalatorsteuerclip (100) zu verwendende aktualisierte Konfigurationsdaten zu bestimmen und die aktualisierten Konfigurationsdaten der Konfigurationskommunikationsschnittstelle (312) bereitzustellen.

13. Inhalatorsteueranordnung (300) gemäß Anspruch 12, wobei die Auswerteeinheit (314) des Konfigurationsgeräts (310) ausgebildet ist
- eintreffenden Sensorausgangssignalen Zeitstempel zuzuweisen und während einer vorbestimmten Überwachungszeitspanne ein Luftverschmutzungsprofil (400) als Funktion der Zeit zu bestimmen und zu speichern, das indikativ für eine Konzentration wenigstens einer Gaskomponente oder Partikel in der Umgebungsatmosphäre ist.

14. Inhalatorsteueranordnung (300) gemäß Anspruch 13, wobei die Auswerteeinheit (314) des Konfigurationsgeräts (310) ausgebildet ist, die aktualisierten Konfigurationsdaten in Abhängigkeit von dem Luftverschmutzungsprofil (400) zu bestimmen.

## Revendications

1. Étui de commande d'inhalateur (100, 200) comprenant
- un boîtier (102) comprenant un réceptacle pour recevoir un inhalateur ;
- une unité de capteur (110) disposée dans le boîtier et comprenant au moins un capteur de pollution de l'air (114, 116, 118), qui est configuré pour effectuer une mesure d'au moins une grandeur indicative d'une concentration d'au moins un composant gazeux ou particulaire dans une atmosphère ambiante et pour fournir au moins un signal de sortie de capteur qui en est indicatif ;
- une unité de commande (112), qui est disposée dans le boîtier (102), reliée à l'unité de capteur (110) et qui est configuré pour déclencher l'unité de capteur (110) pour effectuer la mesure ;
- une unité de communication (122) qui est agencée dans le boîtier et comprend une interface de communication (124) vers un dispositif de configuration externe, et qui est configurée pour recevoir via l'interface de communication des données de configuration indiquant un ou plusieurs points d'échantillonnage dans le temps pour déclencher la réalisation de la mesure et d'au moins une valeur de référence pour la composition de l'atmosphère ambiante ; et
- une unité d'avertissement (120) agencée dans le boîtier (102) et configurée pour recevoir le signal de sortie du capteur et la valeur de référence et pour comparer le signal de sortie du capteur avec la valeur de référence et pour fournir un signal d'avertissement lors de la détermination que la concentration de l'au moins un composant gazeux ou particulaire dans l'atmosphère ambiante dépasse la valeur de référence ; dans lequel
- l'unité de commande (112) reçoit les données de configuration et est configurée pour déclencher l'exécution de la mesure à des points d'échantillonnage configurables dans le temps à l'aide des données de configuration.

2. Étui de commande d'inhalateur (100, 200) selon la revendication 1, dans lequel
- l'unité de commande (112) est configurée pour déclencher l'unité de capteur (110) pour effectuer la mesure à une pluralité de points d'échantillonnage dans le temps dans un intervalle de temps de seuil configurable ; et dans lequel
- l'unité d'avertissement (120) est configurée pour fournir le signal d'avertissement lors de la détermination que la concentration de l'au moins un composant gazeux ou particulaire dans l'atmosphère ambiante dépasse la valeur de référence à tous les points d'échantillonnage dans le temps dans l'intervalle de temps seuil configurable.

3. Étui de commande d'inhalateur (100, 200) selon la revendication 1 ou 2,
- l'unité de communication (122) est configurée pour recevoir via l'interface de communication (124) une pluralité de valeurs de référence pour la composition de l'atmosphère ambiante qui sont associées à différents composants ou particules gazeux ;
- l'unité d'avertissement (120) est configurée pour fournir le signal d'avertissement lors de la détermination du fait qu'une combinaison d'au moins deux des valeurs de référence est dépassée par la concentration de l'au moins un composant gazeux ou particulaire dans l'atmosphère ambiante.

4. Étui de commande d'inhalateur (100, 200) selon l'une des revendications précédentes, dans lequel le capteur de pollution de l'air (114, 116, 118) est configuré pour effectuer une mesure d'une quantité indicative d'une concentration de dioxyde d'azote ou de dioxyde de soufre.

5. Étui de commande d'inhalateur (100, 200) selon l'une des revendications précédentes, comprenant en outre au moins un capteur ambiant qui est configuré pour effectuer une mesure d'au moins une grandeur indicative d'une température de l'atmosphère ambiante, d'une humidité relative. de l'atmosphère ambiante ou une pression de l'atmosphère ambiante.

6. Étui de commande d'inhalateur (100, 200) selon l'une des revendications précédentes, comprenant en outre un affichage (214), qui reçoit le signal d'avertissement et est configuré pour fournir, lors de la réception du signal d'avertissement, une représentation graphique indiquant que la concentration de l'au moins un composant ou des particules gazeux dans l'atmosphère ambiante dépasse la valeur de référence.

7. Étui de commande d'inhalateur (100, 200) selon l'une des revendications précédentes, dans lequel l'unité d'avertissement comprend une unité d'émission de lumière (126, 212) configurée pour émettre le signal d'avertissement en tant que signal lumineux dans l'une respective d'une pluralité de couleurs de lumière distinctes, et pour déterminer la couleur de lumière à émettre en utilisant une quantité de la concentration mesurée de l'au moins un composant gazeux ou particulaire dans l'atmosphère ambiante.

8. Étui de commande d'inhalateur (100, 200) selon l'une des revendications précédentes, dans lequel
- l'unité de commande (112) est configurée pour déclencher l'unité de communication (122) pour effectuer une séquence d'échange de configuration (125) avec le dispositif de configuration externe, la séquence d'échange de configuration (125) comprenant la retransmission du signal de sortie du capteur vers le dispositif de configuration externe et recevoir en réponse des données de configuration mises à jour du dispositif de configuration externe ; et dans lequel
- l'unité de communication (122) est configurée pour effectuer la séquence d'échange de configuration et transmettre les données de configuration mises à jour reçues à l'unité de commande (112).

9. Étui de commande d'inhalateur (100, 200) selon la revendication 8, comprenant en outre
- une unité de balayage configurée pour balayer une section de surface du récipient pour détecter un code graphique identifiant un type de médicament dans le récipient de stockage et fournir un signal de sortie de l'unité de balayage indicatif de celui-ci ;
dans lequel la séquence d'échange de configuration comprend en outre la fourniture du signal de sortie de l'unité de balayage au dispositif de configuration externe.

10. Étui de commande d'inhalateur (100, 200) selon la revendication 6, dans lequel l'unité de communication (122) est en outre configurée pour recevoir via l'interface de communication des données de recommandation de médicament indiquant un dosage de médicament recommandé et pour fournir les données de recommandation de médicament à l'affichage pour sortie à un utilisateur.

11. Étui de commande d'inhalateur (100, 200) selon l'une des revendications précédentes, comprenant en outre un capteur d'actionnement, qui est configuré pour détecter l'actionnement d'un élément d'actionnement d'inhalation sur un inhalateur externe (204) dans un état inséré et pour fournir un signal d'actionnement indicatif de celui-ci, dans lequel l'unité de commande (112), lors de la réception du signal d'actionnement, est configurée pour déclencher l'unité de capteur (110) pour effectuer la mesure.

12. Agencement de commande d'inhalateur (300) comprenant
- au moins une étui de commande d'inhalateur (100) selon la revendication 1;
- un dispositif de configuration (310) pouvant être connecté en communication à l'unité de communication (122) de l'étui de commande d'inhalateur (100) et comprenant
- une interface de communication de configuration (312), qui est configurée pour recevoir un ou plusieurs parmi les signaux de sortie de capteur de l'unité de communication (122) de l'étui de commande d'inhalateur (100) et pour fournir des données de configuration mises à jour à l'unité de communication (122) de l'étui de commande de l'inhalateur ;
- une unité d'évaluation (314) configurée pour déterminer, à l'aide du ou des signaux de capteur reçus, des données de configuration mises à jour à utiliser par l'étui de commande d'inhalateur (100) et pour fournir les données de configuration mises à jour à l'interface de communication de configuration (312).

13. Agencement de commande d'inhalateur (300) selon la revendication 12, dans lequel l'unité d'évaluation (314) du dispositif de configuration (310) est configurée
- pour attribuer des horodatages aux signaux de sortie de capteur entrants et pour déterminer et stocker un profil de pollution (400) indicatif de la concentration de l'au moins un composant gazeux ou particulaire dans l'atmosphère ambiante en fonction du temps pendant une période de temps de surveillance prédéterminée.

14. Agencement de commande d'inhalateur (300) selon la revendication 13, dans lequel l'unité d'évaluation (314) du dispositif de configuration (310) est configurée pour déterminer les données de configuration mises à jour en fonction du profil de pollution (400).
